# EUROPEAN PATENT SPECIFICATION

(11) **EP 4 058 040 B1**
(45) Date of publication and mention of the grant of the patent: **07.01.2026**
(21) Application number: 20820531.0
(22) Date of filing: 11.11.2020
(51) Int. Cl.: A61K 36/63, A61K 36/73, A61K 31/045, A61P 3/06, A23L 33/00, A61K 9/00, A61K 9/20

(54) **COMPOSITION FOR REDUCING CARDIOVASCULAR RISK**
ZUSAMMENSETZUNG ZUR HERABSETZUNG VON KARDIOVASKULÄREM RISIKO
COMPOSITION POUR RÉDUIRE LE RISQUE CARDIOVASCULAIRE

(30) Priority: 11.11.2019 IT 201900020808
(43) Date of publication of application: 21.09.2022
(73) Proprietor: Neilos S.r.l., 80063 Piano di Sorrento (NA) (IT)
(72) Inventor: DI MAIO, Umberto, 80065 Sant'Agnello (NA) (IT)
(74) Representative: Di Giovine, Paolo
(86) International application number: PCT/IB2020/060621
(87) International publication number: WO 2021/094948

(56) References cited:
- WO-A1-2005/000041
- US-A1- 2017 020 948
- GOUNI-BERTHOLD I ET AL: "Policosanol: Clinical pharmacology and therapeutic significance of a new lipid-lowering agent", AMERICAN HEART JOURNAL, ELSEVIER, AMSTERDAM, NL, vol. 143, no. 2, 1 February 2002 (2002-02-01), pages 356 - 365, XP002263555, ISSN: 0002-8703, DOI: 10.1067/MHJ.2002.119997
- GIAN CARLO TENORE ET AL: "Annurca ( Malus pumila Miller cv. Annurca) apple as a functional food for the contribution to a healthy balance of plasma cholesterol levels: results of a randomized clinical trial", JOURNAL OF THE SCIENCE OF FOOD AND AGRICULTURE, 1 September 2016 (2016-09-01), GB, XP055325245, ISSN: 0022-5142, DOI: 10.1002/jsfa.8016
- SATO ET AL: "Anti-hyperglycemic activity of a TGR5 agonist isolated from Olea europaea", BIOCHEMICAL AND BIOPHYSICAL RESEARCH COMMUNICATIONS, ELSEVIER, AMSTERDAM, NL, vol. 362, no. 4, 15 September 2007 (2007-09-15), pages 793 - 798, XP022249510, ISSN: 0006-291X, DOI: 10.1016/J.BBRC.2007.06.130
- BENNANI-KABCHI N ET AL: "Effects of Olea europea var. oleaster leaves in hypercholesterolemic insulin-resistant sand rats", THERAPIE, SOCIETE FRANCAISE DE PHARMACOLOGIE ET DE THERAPEUTIQUE, FR, vol. 54, no. 6, 1 November 1999 (1999-11-01), pages 717 - 723, XP009099955, ISSN: 0040-5957
- XU ET AL: "Dietary octacosanol reduces plasma triacylglycerol levels but not atherogenesis in apolipoprotein E-knockout mice", NUTRITION RESEARCH, ELSEVIER INC, XX, vol. 27, no. 4, 10 April 2007 (2007-04-10), pages 212 - 217, XP022025603, ISSN: 0271-5317, DOI: 10.1016/J.NUTRES.2007.01.015
- HUANG SHAN ET AL: "Hypolipidemic and Antioxidant Effects of Malus toringoides (Rehd.) Hughes Leaves in High-Fat-Diet-Induced Hyperlipidemic Rats", JOURNAL OF MEDICINAL FOOD, MARY ANN LIEBERT, LARCHMONT, NY, US, vol. 20, no. 3, 28 February 2017 (2017-02-28), pages 258 - 264, XP009520794, ISSN: 1096-620X, [retrieved on 20170228], DOI: 10.1089/JMF.2016.3778
- CUMAOGLU AHMET ET AL: "Effects of olive leaf polyphenols against H2O2 toxicity in insulin secreting beta-cells", ACTA BIOCHIMICA POLONICA, POLSKIE TOWARZYSTWO BIOCHEMICZNE, PL, vol. 58, no. 1, 1 January 2011 (2011-01-01), pages 45 - 50, XP009520795, ISSN: 0001-527X
- CERVANTES-CARDOZA VERONICA ET AL: "Antioxidant activity of seed extracts from three apple cultivars (Malus domestica Borkh - Rosaceae-)", BOLETÍN LATINOAMERICANO Y DEL CARIBE DE PLANTAS MEDICINALES Y AROMÁTICAS, SOCIEDAD LATINOAMERICANA DE FITOQUÍMICA, vol. 9, no. 6, 31 October 2010 (2010-10-31), pages 446 - 456, XP009520798, ISSN: 0717-7917

## Description

The present invention relates to a composition for reducing plasma cholesterol levels and thus for reducing cardiovascular risk.

Hypercholesterolaemia is a pathological condition characterised by the presence of high blood levels of lipids, such as cholesterol, triglycerides or phospholipids. It is considered to be the major risk factor for atherosclerosis and cardiovascular disease given that the excessive increase in low-density lipoprotein (LDL) levels has been associated with an increased coronary risk. Therefore, reducing LDL levels seems to be the best approach to prevent cardiovascular disorders.

Hypercholesterolaemia is caused by many factors. Familial hypercholesterolaemia forms are characterised by a hyperproduction of cholesterol at hepatic level, in most cases due to a mutation of the gene that codes for the LDL cellular receptor. The latter, not functioning properly, fails to bind circulating LDL particles and remove them from the blood stream.

In developed countries, dyslipidaemias are very often linked to the so-called metabolic syndrome and they are thus due to bad eating habits and unhealthy lifestyles. Besides high LDL levels and low levels of high-density lipoproteins (HDL), this type of dyslipidaemia is also characterised by obesity, insulin resistance, hypertension and type 2 diabetes. Epidemiological studies have also shown the involvement of other factors such as: increased plasma fibrinogen, coagulation factors, platelet activation and changes in the oxidative state of LDLs.

The primary pharmacological strategy used for the control of dyslipidaemias consists in the administration of statins alone or in combination with other active ingredients such as cholesterol absorption inhibitors or PCSK9 inhibitors, an enzyme that regulates the degradation of LDL receptors.

However, this kind of therapy reveals considerable drawbacks. First and foremost, statins may have side effects in the muscles and liver such as, for example myopathies, rhabdomyolysis (in the most severe cases), impaired liver function and increased transaminases.

Furthermore, not all patients are suitable for such treatment given that, while on the one hand it is an almost obligatory choice for patients with a high or very high cardiovascular risk, on the other there is a significant number of subjects with mild or moderate cardiovascular risk and they have LDL levels close to normal. A simple change of lifestyle is generally recommended to the latter.

In addition, cases of treatment-resistant subjects and cases of intolerant subjects have been reported among patients receiving statin treatment.

In statin-resistant patients, there is generally inadequate or non-reduction in LDL-cholesterol levels. However, this occurs alongside the onset of side effects. The main cause of resistance phenomena lies in the polymorphism of HMG-CoA reductase, of P-glycoprotein, of cholesterol 7 a-hydroxylase, apolipoprotein E, lipoprotein A or LDL receptor.

The use of statins is not recommended for intolerant patients due to the sudden onset of side effects in their most severe form.

WO 2005/000041 A1 discloses combinations of vasoprotective agent and formulations containing them, including polycosanols, for the treatment of vascular injuries induced by an excess of lipids.

US 2017/020948 A1 concerns compositions comprising apple and olive extracts and their synergistic effects in the regulation of oxidative stress in the treatment of high cholesterol levels, metabolic syndrome, arterial hypertension, and atherosclerosis.

Sato et al: "Anti-hyperglycemic activity of a TGR5 agonist isolated from Olea europaea*"* relates to the anti-hyperglycemic activity of a TGR5 agonist isolated from *Olea europaea* and to the effects thereof in improving metabolic disorders.

Bennani-Kabchi et al: "Effect of Olea europea var. oleaster leaves in hypercholesterolemic insulin-resistant rats" reports on the effects of *Olea europaea var. oleaster* leaves in hypercholesterolemic insulin-resistant sand rats, demonstrating that oleaster leaves possess at least two active compounds useful in the treatment of hypercholesterolemia and diabetes.

Xu et al: "Dietary octacosanol reduces plasma triacylglycerol levels but not atherogenesis in apolipoprotein E-knockout mice*"* reports that dietary octacosanol reduces plasma triacylglycerol levels but not atherogenesis in apolipoprotein E-knockout mice.

Huang Shan et al: "Hypolipidemic and Antioxidant Effects of Malus toringoides (Rehd.) Hughes Leaves in High-Fat-Diet-Induced Hyperlipidemic Rats*"* concerns the hypolipidemic and antioxidant effects of *Malus toringoides* (MT) in high-fat-diet-induced hyperlipidemic rats, concluding that MT exerts considerable hypolipidemic and antioxidant properties.

Cumaoglu Ahmet et al: "Effects of olive leaf polyphenols against H2O2 toxicity in insulin-secreting beta-cells*"* reports on the modulatory effects of polyphenolic constituents of olive leaves (*Olea europaea*) in the case of chronic hyperglycemia and diabetes.

Cervantes-Cardoza Veronica et al: "Antioxidant activity of seed extracts from three apple cultivars (Malus domestica Borkh - Rosaceae)*"* reveals that oily and aqueous extracts from apple seeds (*Malus domestica*) show protection against low-density lipoprotein (LDL) oxidation, favorable in the treatment of atherosclerosis.

Therefore, in the art there arises the need for alternative strategies as support in cases of resistant or intolerant patients or so as to ensure a form of prevention in all cases where hypercholesterolaemia represents a low or moderate risk factor.

The invention is set out in the appended set of claims.

An object of the present invention is to provide an alternative treatment with respect to those known in the art for the treatment of hypercholesterolaemia in patients resistant or intolerant to statins and/or for the prevention of hypercholesterolaemia in patients with a risk factor for mild or moderate cardiovascular disorders.

Such object is achieved by the present invention given that it relates to a composition according to claim 1 and to the use thereof according to claims 7 and 9. Preferred embodiments of the invention form the object of the dependent claims.

In particular, herein provided is a composition comprising an extract of a plant belonging to the genus Malus, an extract of Olea Europaea and policosanols.

Advantageously, the composition of the invention has shown a synergistic effect between the components allowing a reduction in hypercholesterolemia, an increase in HDL levels, a reduction in lipid peroxidation and intestinal cholesterol absorption.

In an embodiment, the composition of the invention has shown a synergistic effect between the components allowing to obtain a decrease in the total cholesterol value, preferably at an amount comprised from 5% to 40%; more preferably comprised from 10% to 35%; even more preferably from 15% to 30%, for example about 20% or about 25%, with respect to the initial values before the treatment of the same subject (the % values refer to before and after the treatment of the same subject as explained hereinafter).

In an embodiment, the composition of the invention has shown a synergistic effect between the components allowing to obtain a decrease in the LDL value, preferably at an amount comprised from 10% to 40%; more preferably comprised from 15% to 35%; even more preferably from 20% to 30%, for example about 25%, with respect to the initial values before the treatment of the same subject (the % values refer to before and after the treatment of the same subject as explained hereinafter).

In an embodiment, the composition of the invention has shown a synergistic effect between the components allowing to obtain an increase in the HDL value, preferably at an amount comprised in the range from 15% to 40%; more preferably comprised from 20% to 35%; even more preferably from 25% to 30%, with respect to the initial values before the treatment of the same subject (the % values refer to before and after the treatment of the same subject as explained hereinafter).

The composition of the present invention may also contain other pharmaceutically acceptable functional substances and excipients.

Suitable pharmacologically acceptable excipients are those commonly known to the man skilled in the art for the preparation of pharmaceutical compositions for oral administration such as solutions, suspensions, powders, tablets, capsules, etc.

By way of non-limiting example, said pharmaceutically acceptable excipients can be selected from diluents (for example dibasic calcium phosphate, lactose, microcrystalline cellulose and cellulose derivatives), thickeners (for example hydroxypropyl methylcell ulose gums and other cellulose derivatives), sweeteners (for example sorbitols, mannitols and other polyols, acesulfame K, aspartame, cyclamates, saccharin, sucralose), lubricants (for example magnesium stearate, stearic acid, waxes), dispersants, surfactants (for example sodium lauryl sulfate and polysorbates), flavouring agents, adsorbers (for example silica gel, talc, starch, bentonite, kaolin), glidants and anti-stick agents (for example talc, colloidal silica, corn starch), dyes, antioxidants, binders (for example gums, starch, gelatine, cellulose derivatives, sucrose, sodium alginate), disintegrants (starch, microcrystalline cellulose, alginic acid, crospovidone), plasticisers (for example ethyl cellulose and other cellulose derivatives, acrylates and methacrylates), thickeners, emulsifiers, humectants, wetting agents, preservatives, chelating agents and mixtures thereof.

The composition subject of the present invention is preferably a solid or liquid pharmaceutical composition for oral use. In an embodiment, the composition subject of the present invention is in the form of powder, oral soluble powder, granulate, hard capsule, soft-gel capsule, tablet, sachet, solution, suspension, syrup, gel stick or bottle.

In an embodiment, the extract of a plant belonging to the genus Malus may be present at an amount comprised between 1 mg and 5500 mg, preferably between 10 mg and 3000 mg, even more preferably between 50 mg and 1000 mg; preferably for single dose unit.

The extract of Olea Europaea may be present at an amount comprised between 1 mg and 8000 mg, preferably between 2.5 mg and 4000 mg, even more preferably between 10 mg and 1000 mg; preferably for single dose unit.

Policosanols may be present at an amount comprised between 0.1 mg and 1000 mg, preferably between 1 mg and 800 mg, even more preferably between 2 mg and 200 mg; preferably for single dose unit.

The composition subject of the present invention is for use in a method for the treatment of hypercholesterolaemia in patients resistant or intolerant to statins.

The composition subject of the present invention is for use in a method for the prevention of hypercholesterolaemia in patients with a risk factor for mild or moderate cardiovascular disorders.

The composition subject of the present invention is for use in a method for the treatment of hypercholesterolaemia in patients resistant or intolerant to statins and/or in a method for the prevention of hypercholesterolaemia in patients with a risk factor for mild or moderate cardiovascular disorders.

### GLOSSARY

The terms used in the present description are as generally understood by the man skilled in the art, unless otherwise indicated.

In the context of the present description, the term "extract" is used to indicate any product related to a plant drug including all products derived from mechanical treatments (pulverisation, crushing, mixing and/or other methods) or from extract-based treatments (solvent extraction, distillation, and/or other specific methods) conducted on a drug.

The extract of a plant belonging to the genus Malus can be an extract of the fruit, seed, gem, bark of the roots, leaves.

The extract of Olea Europaea is an extract of the fruit, leaves, gem, suckers, oleum.

### ANNURCA APPLE

There are several botanical species belonging to the genus Malus, one of the most interesting being the annurca apple.

Malus pumila var. annurca or *Malus domastica* var.annurca is the only apple variety native to the south of Italy, mainly from the Campania region, between Caserta and Benevento. The annurca apple has a pulp rich in juice, compact, crunchy and characterised by the typical sour taste that distinguishes it from the other apple varieties.

Apples have always been attributed health properties. Today it is known that the benefits of apple consumption can be attributed to the polyphenolic content of the fruit consisting of quercetin, epicatechin, catechin, procyanidins, anthocyanins, dihydrochalcones and phenolic acids. Although the components are essentially the same, the specific quantities vary among the different apple varieties. The annurca apple has proved to be particularly interesting because of its very high polyphenolic content, which is entirely characteristic.

Polyphenols can generally be in the form of dimers, trimers, tetramers, pentamers and high molecular weight oligomers. In the annurca apple the polyphenol fraction is mainly rich in procyanidins, in particular procyanidin B2, and oligomers formed by a number of monomers comprised between 6 and 8.

Apple extract is known to have chemoprotective properties. As a matter of fact, numerous studies have shown that fresh apple extract is capable of inhibiting cell growth in various types of cancer, such as colon and liver cancer, in a dose-dependent manner. Furthermore, numerous products based on polyphenolic apple extracts proposed for body weight control, as cosmetics and for their anti-inflammatory action are commercially available.

Recent studies show that some polyphenols contained in the apple have a beneficial effect in patients with metabolic syndrome. In particular, phlorizin is the main responsible for the blood sugar lowering action. In vitro and in vivo studies show the blood sugar lowering effect highlighting that the compound in question induces glucosuria and blocks intestinal glucose absorption.

The lipid-lowering effect is mainly due to procyanidins.

The complex mechanism of action of annurca apple polyphenols has been brought to light by several in vitro studies. The beneficial effect on hypercholesterolaemia is due to the combined action of dimeric (particularly procyanidin B2) and oligomeric procyanidins. Both of these fractions of the annurca polyphenolic complex are responsible for the lipid-lowering effect but with different mechanisms of action. In particular, high molecular weight oligomers are not absorbed at the intestinal level where they accumulate and act locally to reduce cholesterol levels by acting in a similar manner to b-cyclodextrins and forming micelle-like complexes that incorporate cholesterol and prevent the absorption thereof. On the other hand, dimers are rapidly absorbed in the intestinal tract and quickly reach the liver where they are indirectly involved in the increase of LDL receptors.

Therefore, dimeric procyanidins are capable of reducing LDL cholesterol levels as effectively as statins and, unlike statins which have no effect on HDL, form stable complexes with apolipoprotein A1, the major structural protein component of HDL and increase the reverse cholesterol pathway and, thus, levels of eliminated cholesterol.

A preliminary clinical trial in 250 patients with moderate hypercholesterolaemia evaluated the effect that the intake of two annurca apples daily has on the lipid profile of the subjects considered compared to that of other apple varieties. The results of the trial showed that, with respect to other apple varieties highlighted, annurca apple is responsible for a significant reduction in total cholesterol and LDL cholesterol levels and the increase in HDL levels.

Based on these data, a 16-week single-centre, double-blind, placebo-controlled clinical trial on 250 subjects with mild and moderate hyperlipidaemia was established. The subjects selected for the trial (116 men and 134 women) were 30 to 83 years of age and had the following baseline cholesterol levels:
total cholesterol 214 - 254 mg/dL;
LDL cholesterol 150 - 205 mg/dL;
HDL cholesterol 30 - 43 mg/dL.

Trial participants were subjected to a first 4-week trial phase, in which a placebo was administered, followed by an 8-week phase two, in which two capsules of polyphenol extract of annurca apples containing 400 mg each were administered daily, ending with 4 weeks of follow-up. There were no changes in total, LDL and HDL cholesterol levels in the placebo phase. The results recorded in phase 2 showed, as early as one month of treatment, a reduction in total and LDL cholesterol levels by 24.9% and 37.5%, respectively, and an increase in HDL by 49.2%.

Thus, the reported data show that the polyphenolic extract of annurca apple represents a valid alternative to the therapeutic treatment for the control of hypercholesterolaemia in subjects with moderate impairment of the lipid profile.

### POLICOSANOLS

The term policosanols refers to a mixture of long-chain aliphatic alcohols present in beeswax, in potatoes, in rice bran and, above all, in sugar cane (*Saccharum officinanim*). The main component of policosanols is octacosanol, present for about 60% - 70%. They also contain aliphatic alcohols such as triacontanol (about 12%), hexacosanol (7%), and the remaining part consists of tetracosanol, heptacosanol, nonacosanol and other components in a smaller amount.

Polycosanols have been extensively studied for their cholesterol-lowering properties and, thus, there is a large number of pre-clinical and clinical trials that prove their beneficial effects on the lipid profile. The use of policosanols is useful in the present invention given that it reduces hypercholesterolaemia with a pharmacological profile similar to that of statins without the onset of side effects. The effect on hypercholesterolaemia is due to the action of the aforementioned alcohols on the hyd roxymethy I gl utary I-CoA reductase (HMG-CoA reductase) enzyme which converts hydroxymethylglutaryl-CoA into mevalonate and which is the limiting enzyme in the hepatic cholesterol biosynthesis process. Unlike statins, policosanols do not competitively inhibit enzyme activity but block cholesterol synthesis in an earlier stage. As a matter of fact, some studies have shown that in fibroblasts, policosanols depress the expression of HMG-CoA reductase in a concentration-dependent manner, inhibiting the transcription of the gene that codes for this enzyme and simultaneously increasing the degradation thereof. Besides the aforementioned mechanism, policosanols seem effective in improving the LDL metabolism by increasing the binding,
uptake, and degradation of the latter. Several clinical trials have evaluated the cholesterol-lowering potential of polycosanols. In all studies evaluated, treatment with policosanols decreased the levels of total cholesterol, LDL cholesterol and LDL/HDL ratio.

In addition, an increase in HDL cholesterol has also been recorded in some studies. Other trials have compared the efficacy of policosanols to that of some statins (simvastatin, pravastatin, atorvastatin and lovastatin), showing that the alcohol mixture administered has an efficacy comparable to the pharmacological treatment, without side effects.

An in vivo study on rabbits investigated the effect of policosanols on the lipid profile of the animals involved in the study. The rabbits were divided into two groups and subjected to treatment:
- Control group (n = 15): diet rich in fat for 30 days;
- Treated group (n = 15): diet rich in fats and 50 mg/kg of policosanols for 30 days.

Plasma levels of total cholesterol and LDL were evaluated at the end of the treatment period. The results reported reveal that the treatment prevents the increase in total cholesterol and LDL cholesterol with respect to the control. Tests conducted on liver tissues taken after animal sacrifice have shown that the treatment carried out reduces plasma cholesterol levels by inhibiting hepatic biosynthesis.

The positive effects of policosanols on hyperlipidaemia are confirmed by a randomised, double-blind clinical trial in 437 patients with hypercholesterolaemia and other coronary risk factors. The identified subjects were divided into two groups:
- Placebo group;
- Treated group: administration of 5 mg of policosanols per day for 12 months followed by administration of 10 mg of policosanols per day for another 12 months.

At the end of 24 months of treatment, treatment with 5 mg and 10 mg of policosanols has been shown to reduce LDL plasma level by 18.2% and 25.6%, the total cholesterol level by 13% and 17.4%, respectively, and increase the HDL levels by 15.5% and 28.4%, respectively. The study did not reveal the onset of any side effects. Therefore, policosanols are safe, well tolerated and effective in reducing hypercholesterolemia and improving the overall lipid picture.

Policosanols have other properties useful to the health of the cardiovascular system. Besides the improvement of the lipid profile, the clinical benefits of policosanols seem to be a consequence of the favourable impact that these substances have on vascular functions and on platelets. They have a major antiplatelet action that prevents possible formation of thrombi, which may cause myocardial infarction, cerebral ischemia and arterial insufficiency. Policosanols have also been shown to reduce the proliferation of vascular smooth muscle cells by preventing the reduction of vessel diameter and, therefore, blood flow. These substances have also been shown to reduce the oxidation of LDL lipoproteins, which is the major factor behind the development of atherosclerotic phenomena.

### OLEA EUROPAEA

*Olea Europaea,* commonly referred to as olive tree, is an evergreen fruit tree belonging to the family of Oleaceae. It lives for a very long time, it can become millenary and reach heights of 15 - 20 metres. It is one of the oldest trees cultivated in the Mediterranean region and it has been used in the food industry since ancient times. Its fruits, the olives, are used for the extraction of olive oil while the standardised extracts of leaves are used for medicinal purposes due to the antidyslipidemic, vasodilating, hypotensive and antiphlogistic properties thereof. A large number of scientific evidence highlights the role of Olea Europaea in the prevention and management of various diseases, including those at the cardiovascular level. In traditional medicine, decoctions, infusions and essential oil of Olea Europaea were used to relieve all types of diseases: urinary infections, asthma, diarrhoea, constipation, high blood pressure, intestinal disorders, rheumatisms, inflammatory conditions, diabetes and gout. Several studies highlight the beneficial role that phenolic extract of Olea Europaea has in many types of cancer. Studies also show the antioxidant action of Olea Europaea and the positive role thereof in controlling blood sugar and lipid profile. The beneficial effects of Olea Europaea can be attributed to its secondary metabolites. Chemical analysis results show that Olea Europaea contains flavonoids, flavonoid glycosides, flavanones, iridoids, secoiridoids, triterpenes, biophenols, benzoic acid derivatives, sterols and xylitol. In particular, the phenolic compound oleuropein is considered the main responsible for the characteristic therapeutic effects and numerous studies have shown that oleuropein is effective as an antioxidant, anti-inflammatory, anti-atherogenic, anti-cancer, antimicrobial and antiviral agent. Olea Europaea is considered a valid natural remedy for the control of hypercholesterolaemia and for the reduction of atherosclerotic risk. Data from numerous in vivo studies show that extracts of Olea Europaea leaves are effective in reducing plasma levels of total, LDL, VLDL cholesterol, triglycerides and increasing HDL levels, reducing blood pressure and preventing LDL oxidation. The ethanolic extract of Olea Europaea leaves has also shown potent antioxidant actions.

The data obtained from an in-silico study are very interesting given that they show that some phenolic compounds of Olea Europaea, and especially rutin, have a high binding affinity for the active site of the HMG-CoA reductase enzyme. Therefore, such data are promising as they predict a possible inhibitory effect of the phenolic extract on the enzyme.

An in vivo study evaluated the lipid-lowering and anti-atherosclerotic properties of the ethanolic extract and of the aqueous extract of Olea Europaea leaves. 40 Wistar rats selected for the study were divided into eight groups and subjected to treatment for eight weeks as follows:
- Group 1 control: rats subjected to said standard diet (SCD);
- Group 2: SCD diet and treatment with Atorvastatin 20 mg/kg;
- Group 3: SCD diet and treatment with *Olea Europaea* extract 50 mg/kg;
- Group 4: SCD diet and treatment with *Olea Europaea* extract 100 mg/kg;
- Group 5: rats subjected to a high-fat diet (HFD);
- Group 6: HFD diet and treatment with Atorvastatin 20 mg/kg;
- Group 7: HFD diet and treatment with *Olea Europaea* extract 50 mg/kg;
- Group 8: HFD diet and treatment with *Olea Europaea* extract 100 mg/kg.

The lipid profile of the animals was analysed at the end of the eight weeks. In animals subjected to a high-fat diet, there is a deterioration of the total lipid profile, with an increase in cholesterol and LDL. Plasma lipid levels decreased significantly with both concentrations used. The levels of antioxidant enzymes and markers of lipid peroxidation were measured on the isolated hearts of mice subjected to treatment. Rats on HFD diet showed a remarkable increase in atherosclerotic index. This effect is reversed following treatment with the extracts. A clinical trial in 33 volunteers with hypercholesterolaemia (TC > 200 mg/dL) evaluated the antioxidant and anti-atherosclerotic effect of the phenolic extract of Olea Europaea. The trial participants underwent a 2- week study period, during which they took a common olive oil, followed by a 3-week period in which they took 25 mL Olea Europaea oil enriched with the phenolic component thereof. Treatment with enriched oil has shown a significant improvement in HDL composition with an increase in antioxidant content. In particular, an increase in the content of a-tocopherol, the main antioxidant effective in combating lipid peroxidation, was observed. Therefore, the Olea Europaea oil enriched with the phenolic component is promising in improving the composition profile of HDL and in reducing cardiovascular risk.

The composition of the invention is a valid aid in reducing hypercholesterolaemia, the major cardiovascular risk factor, thanks to the combined action of the components present, which act at different levels.

Thus, according to a further aspect of the present invention provided for is a composition (formulation of the present invention) comprising an extract of a plant belonging to the genus Malus, an extract of Olea Europaea and policosanols for use in the form of a medical device, food supplement, nutraceutical, dietary or nutritional composition, food product, beverage, food, medicated food, food for special medical purposes,
or pharmaceutical composition or even in the form of a simple, complementary, complete feed intended for particular nutritional or medicated purposes, or pharmaceutical composition, in particular for reducing plasma cholesterol levels and cardiovascular risk both in humans and animals.

Without being bound by theory, the inventors believe that the synergistic effect of the composition of the invention stems from the activities of the components thereof.

Annurca apple extract reduces plasma cholesterol levels by increasing LDL receptors and reducing intestinal absorption, while increasing HDL levels.

Policosanols reduce LDL levels by reducing hepatic biosynthesis thereof, they increase HDL and they decrease vascular oxidation.

*Olea Europaea* extract enhances the action of policosanols and contributes toward reducing plasma cholesterol levels, acting on the liver and the lipid peroxidation phenomena increasing the antioxidant component of HDL.

The action of the individual components and of the combination subject of the present invention is evaluated by means of in vitro and/or in vivo tests.

In vitro tests on murine or human cells may be useful to evaluate the antioxidant action of the formulation in question. The cells were grown in complete DMEM growth medium and kept at 37°C and in a humid atmosphere at 5% di C02 . Following treatment with the extracts of interest, the total RNA extracted from the samples is subjected to RT-PCR to evaluate the levels of expression of pro-inflammatory proteins such as IL-1 b, TNF-a and iNOS.

An in vitro test for assessing oxidative stress levels consists in treating cells with 2,7-dichlorodihydrofluorescein acetate. The fluorescence generated can be measured by means of fluorescence microscope at 529 nm.

An in vitro test may be conducted on a murine or human cell line to evaluate cholesterol uptake. The treated and untreated cells are incubated with labelled cholesterol and then washed to remove the excess. The radioactivity of the solubilised cells is measured at the end of the test.

The effectiveness of the present invention may also be evaluated by means of an in vivo test on experimental animals in compliance with the directives of the European Union and the Ministry of Health and approved by an Ethics Committee. The test is conducted on Apoe T mice which, after an acclimatisation period, are divided into groups and subjected to treatment. Plasma samples are taken at time zero and at established intervals until the end of the treatment period and total cholesterol, LDL cholesterol and HDL cholesterol levels are measured.

Ex vivo tests may be conducted at the end of the treatment period. Therefore, mice are sacrificed and liver, intestinal and arterial tissue samples are taken. The collected and lysed tissues are subjected to Western blot test to evaluate the levels of some proteins of interest.

Levels of HMG-CoA reductase and squalene monooxidase may be evaluated on liver tissue. Levels of pro-inflammatory proteins such as IL-1 b, TNF-a, TLR2, TLR4 and MAP kinase may be evaluated on arterial tissue. The total functionality of the tissue of interest may be evaluated on the intestinal tissue by evaluating the levels of junction proteins such as occludin or JAM-1.

### EXAMPLES

Some examples of daily doses of the active components of the compositions subject of the present invention are now provided for illustrative purposes. Daily doses are intended to be administered in a suitable oral dosage form and divided into one or more dose units such as, for example, a capsule, a tablet or a sachet.

### EXAMPLE 1

| Active ingredient | Amount for single dose unit |
|---|---|
| Annurca Apple | 800 mg |
| Policosanols | 10 mg |
| *Olea Europaea* e.s. | 25 mg |

### EXAMPLE 2

| Active ingredient | Amount for single dose unit |
|---|---|
| Annurca Apple | 500 mg |
| Policosanols | 30 mg |
| *Olea Europaea* e.s. | 100 mg |

### EXAMPLE 3

| Active ingredient | Amount for single dose unit |
|---|---|
| Annurca Apple | 400 mg |
| Policosanols | 20 mg |
| *Olea Europaea* e.s. | 50 mg |

### EXAMPLE 4

| Active ingredient | Amount for single dose unit |
|---|---|
| Annurca Apple | 300 mg |
| Policosanols | 15 mg |
| *Olea Europaea* e.s. | 80 mg |

## Claims

1. A composition comprising or, alternatively, consisting of an extract of a plant belonging to the genus Malus, an extract of Olea Europaea, and policosanols, wherein the extract of a plant belonging to the genus Malus is an extract of Malus pumila var. Annurca.

2. The composition according to claim 1, comprising:
from 1 mg to 5500 mg of an extract of a plant belonging to the genus Malus, preferably for single dose unit;
from 1 mg to 8000 mg of an extract of Olea Europaea and from 0.1 mg to 1000 mg of policosanols, preferably for single dose unit.

3. The composition according to any one of the preceding claims comprising:
from 10 mg to 3000 mg of an extract of a plant belonging to the genus Malus, preferably for single dose unit;
from 2.5 mg to 4000 mg of an extract of Olea Europaea and from 1 mg to 800 mg of policosanols, preferably for single dose unit.

4. The composition according to any one of the preceding claims comprising:
from 50 mg to 1000 mg of an extract of a plant belonging to the genus Malus, preferably for single dose unit;
from 10 mg to 1000 mg of an extract of Olea Europaea, preferably for single dose unit; and
from 2 mg to 200 mg of policosanols, preferably for single dose unit.

5. The composition according to any one of the preceding claims in the form of a solid or liquid composition for oral use.

6. The composition according to claim 5, in a form selected from powder, oral soluble powder, granulate, hard capsule, soft-gel capsule, tablet, sachet, solution, suspension, syrup, gel stick or bottle.

7. The composition according to any one of claims 1 to 6 for use in reducing plasma cholesterol levels and cardiovascular risk in animals.

8. The composition for use according to claim 7, wherein said animals are humans.

9. The composition according to any one of claims 1 to 6 for use in a method for the treatment of hypercholesterolaemia in patients resistant or intolerant to statins.

10. The composition for use according to any one of claims 7 to 9, wherein said composition allows to obtain a decrease in the total cholesterol value, preferably at an amount comprised from 5% to 40%; more preferably from 10% to 35%; even more preferably from 15% to 30%, for example about 20% or about 25%.

11. The composition for use according to any one of claims 7 to 10, wherein said composition allows to obtain a decrease in the LDL value, preferably at an amount comprised from 10% to 40%; more preferably from 15% to 35%; even more preferably from 20% to 30%, for example about 25%.

12. The composition for use according to any one of claims 7 to 11, wherein said composition allows to obtain an increase in the HDL value, preferably at an amount comprised from 15% to 40%; more preferably comprised from 20% to 35%; even more preferably from 25% to 30%.

13. A formulation in the form of a medical device, dietary supplement, nutraceutical, dietary or nutritional composition, food product, beverage, food, medicated food, food for special medical purposes, or pharmaceutical composition, or even in the form of a simple, complementary, complete feed, intended for particular nutritional purposes or medicated, wherein said formulation comprises a composition according to any one of claims 1 to 6 and, optionally, one or more food or pharmaceutical grade technological additives or excipients.

## Patentansprüche

1. Zusammensetzung, umfassend oder alternativ bestehend aus einem Extrakt einer Pflanze, die zu der Gattung Malus gehört, einem Extrakt von Olea Europaea und Policosanolen, wobei der Extrakt einer Pflanze, die zu der Gattung Malus gehört, ein Extrakt von Malus pumila var. Annurca ist.

2. Zusammensetzung nach Anspruch 1, umfassend:
von 1 mg bis 5500 mg eines Extrakts einer Pflanze, die zu der Gattung Malus gehört, vorzugsweise für eine Einzeldosiseinheit;
von 1 mg bis 8000 mg eines Extrakts von Olea Europaea und von 0,1 mg bis 1000 mg Policosanole, vorzugsweise für eine Einzeldosiseinheit.

3. Zusammensetzung nach einem der vorstehenden Ansprüche, umfassend:
von 10 mg bis 3000 mg eines Extrakts einer Pflanze, die zu der Gattung Malus gehört, vorzugsweise für eine Einzeldosiseinheit;
von 2,5 mg bis 4000 mg eines Extrakts von Olea Europaea und von 1 mg bis 800 mg Policosanole, vorzugsweise für eine Einzeldosiseinheit.

4. Zusammensetzung nach einem der vorstehenden Ansprüche, umfassend:
von 50 mg bis 1000 mg eines Extrakts einer Pflanze, die zu der Gattung Malus gehört, vorzugsweise für eine Einzeldosiseinheit;
von 10 mg bis 1000 mg eines Extrakts von Olea Europaea, vorzugsweise für eine Einzeldosiseinheit; und von 2 mg bis 200 mg Policosanole, vorzugsweise für eine Einzeldosiseinheit.

5. Zusammensetzung nach einem der vorstehenden Ansprüche in der Form einer festen oder flüssigen Zusammensetzung für eine orale Verwendung.

6. Zusammensetzung nach Anspruch 5, in einer Form, ausgewählt aus Pulver, oral löslichem Pulver, Granulat, Hartkapsel, Weichgelkapsel, Tablette, Beutel, Lösung, Suspension, Sirup, Gelstift oder Flasche.

7. Zusammensetzung nach einem der Ansprüche 1 bis 6 zur Verwendung bei einem Reduzieren eines Plasmacholesterinspiegels und eines kardiovaskulären Risikos bei Säugetieren.

8. Zusammensetzung zur Verwendung nach Anspruch 7, wobei die Säugetiere Menschen sind.

9. Zusammensetzung nach einem der Ansprüche 1 bis 6 zur Verwendung bei einem Verfahren für die Behandlung von Hypercholesterinämie bei Patienten, die gegenüber Statinen resistent oder intolerant sind.

10. Zusammensetzung zur Verwendung nach einem der Ansprüche 7 bis 9, wobei die Zusammensetzung ermöglicht, eine Senkung des Gesamtcholesterinwerts zu erhalten, vorzugsweise in einer Menge von 5 % bis 40 %; mehr bevorzugt von 10 % bis 35 %; noch mehr bevorzugt von 15 % bis 30 %, zum Beispiel etwa 20 % oder etwa 25 %.

11. Zusammensetzung zur Verwendung nach einem der Ansprüche 7 bis 10, wobei die Zusammensetzung ermöglicht, eine Senkung des LDL-Werts zu erhalten, vorzugsweise in einer Menge von 10 % bis 40 %; mehr bevorzugt von 15 % bis 35 %; noch mehr bevorzugt von 20 % bis 30 %, zum Beispiel etwa 25 %.

12. Zusammensetzung zur Verwendung nach einem der Ansprüche 7 bis 11, wobei die Zusammensetzung ermöglicht, eine Erhöhung des HDL-Werts zu erhalten, vorzugsweise in einer Menge von 15 % bis 40 %; mehr bevorzugt von 20 % bis 35 %; noch mehr bevorzugt von 25 % bis 30 %.

13. Formulierung in der Form eines Medizinprodukts, eines Nahrungsergänzungsmittels, eines Nutrazeutikums, einer diätetischen oder nutritiven Zusammensetzung, eines Lebensmittelprodukts, eines Getränks, eines Lebensmittels, eines medizinisch angereicherten Lebensmittels, eines Lebensmittels für besondere medizinische Zwecke oder einer pharmazeutischer Zusammensetzung oder auch in der Form eines einfachen, ergänzenden, vollständigen Futtermittels, das für besondere Ernährungszwecke bestimmt oder medizinisch angereichert ist, wobei die Formulierung eine Zusammensetzung nach einem der Ansprüche 1 bis 6 und optional einen oder mehrere technologische Zusatzstoffe oder Hilfsstoffe in Lebensmittel- oder pharmazeutischer Qualität umfasst.

## Revendications

1. Composition comprenant ou, en variante, consistant en un extrait d'une plante appartenant au genre Malus, un extrait d'Olea Europaea, et des policosanols, dans lequel l'extrait d'une plante appartenant au genre Malus est un extrait de Malus pumila var. Annurca.

2. Composition selon la revendication 1, comprenant :
de 1 mg à 5500 mg d'un extrait d'une plante appartenant au genre Malus, de préférence pour une unité à dose unique ;
de 1 mg à 8000 mg d'un extrait d'Olea Europaea et de 0,1 mg à 1000 mg de policosanols, de préférence pour une unité à dose unique.

3. Composition selon l'une quelconque des revendications précédentes, comprenant :
de 10 mg à 3000 mg d'un extrait d'une plante appartenant au genre Malus, de préférence pour une unité à dose unique ;
de 2,5 mg à 4000 mg d'un extrait d'Olea Europaea et de 1 mg à 800 mg de policosanols, de préférence pour une unité à dose unique.

4. Composition selon l'une quelconque des revendications précédentes, comprenant :
de 50 mg à 1000 mg d'un extrait d'une plante appartenant au genre Malus, de préférence pour une unité à dose unique ;
de 10 mg à 1000 mg d'un extrait d'Olea Europaea, de préférence pour une unité à dose unique ; et de 2 mg à 200 mg de policosanols, de préférence pour une unité à dose unique.

5. Composition selon l'une quelconque des revendications précédentes sous la forme d'une composition solide ou liquide pour utilisation orale.

6. Composition selon la revendication 5, sous une forme choisie parmi poudre, poudre soluble dans la bouche, granules, capsule dure, capsule molle, comprimé, sachet, solution, suspension, sirop, stick de gel ou flacon.

7. Composition selon l'une quelconque des revendications 1 à 6 pour utilisation dans la réduction de taux de cholestérol plasmatique et de risque cardiovasculaire chez des animaux.

8. Composition pour utilisation selon la revendication 7, dans laquelle lesdits animaux sont des humains.

9. Composition selon l'une quelconque des revendications 1 à 6 pour utilisation dans un procédé destiné au traitement d'hypercholestérolémie chez des patients résistants ou intolérants aux statines.

10. Composition pour utilisation selon l'une quelconque des revendications 7 à 9, dans laquelle ladite composition permet d'obtenir une diminution de la valeur de cholestérol total, de préférence dans une proportion comprise de 5 % à 40 % ; plus préférablement de 10 % à 35 % ; même plus préférablement de 15 % à 30 %, par exemple environ 20 % ou environ 25 %.

11. Composition pour utilisation selon l'une quelconque des revendications 7 à 10, dans laquelle ladite composition permet d'obtenir une diminution de la valeur LDL, de préférence dans une proportion comprise de 10 % à 40 % ; plus préférablement de 15 % à 35 % ; même plus préférablement de 20 % à 30 %, par exemple d'environ 25 %.

12. Composition pour utilisation selon l'une quelconque des revendications 7 à 11, dans laquelle ladite composition permet d'obtenir une augmentation de la valeur HDL, de préférence dans une proportion comprise de 15 % à 40 % ; plus préférablement comprise de 20 % à 35 % ; même plus préférablement de 25 % à 30 %.

13. Formulation sous la forme d'un dispositif médical, d'un complément alimentaire, d'un nutraceutique, d'une composition diététique ou nutritionnelle, d'un produit alimentaire, d'une boisson, d'un aliment, d'un aliment médicamenteux, d'un aliment destiné à des fins médicales spéciales, ou d'une composition pharmaceutique, ou même sous la forme d'un aliment simple, complémentaire, complet, destiné à des fins nutritionnelles particulières ou médicamenteux, dans laquelle ladite formulation comprend une composition selon l'une quelconque des revendications 1 à 6 et, facultativement, un ou plusieurs additifs technologiques ou excipients de qualité alimentaire ou pharmaceutique.
